# EUROPÄISCHE PATENTSCHRIFT

(11) **EP 2 111 896 B1**
(45) Veröffentlichungstag und Bekanntmachung des Hinweises auf die Patenterteilung: **10.08.2016**
(21) Anmeldenummer: 08007709.2
(22) Anmeldetag: 21.04.2008
(51) Int. Cl.: A61N 5/06, A61J 15/00, A61M 27/00

(54) **Vorrichtung für die medizinische Bestrahlung**
Device for medical radiation
Dispositif pour rayonnement médical

(43) Veröffentlichungstag der Anmeldung: 28.10.2009
(73) Patentinhaber: N.V. Nutricia, 2712 HM Zoetermeer (NL); HELTSCHL GmbH, 4707 Schlüßlberg (AT)
(72) Erfinder: Wolkenstörfer, Reinhold, 91077 Neunkirchen (DE); Heltschl, Hermann, 4707 Schlüßlberg (AT); Heltschl, Andreas, 4707 Schlüßlberg (AT)
(74) Vertreter: von Hellfeld, Axel

(56) Entgegenhaltungen:
- WO-A-01/87416
- DE-A1- 4 340 997
- DE-A1-102006 001 736
- DE-U1- 29 611 520
- US-A- 3 131 690
- US-A- 4 248 214
- US-A- 5 588 952
- US-A- 5 879 306
- US-A1- 2002 099 293
- US-A1- 2004 093 044

## Beschreibung

Die Erfindung betrifft eine medizinische Bestrahlungsvorrichtung mit den Merkmalen des Oberbegriffs des Patentanspruchs 1. Eine solche Vorrichtung ist aus der DE 10 2006 001 736 A1 bekannt.

Es ist im Stand der Medizintechnik bekannt, mit im Wesentlichen monochromatischer elektromagnetischer Strahlung eine sogenannte Biostimulation in oberflächennahen Bereichen eines Patienten mit einer Eindringtiefe von bis zu 1 bis 2 cm zu bewirken. Wesentlich für die hier in Rede stehende Biostimulation ist, dass die verwendeten Strahlungsleistungen und Strahlungsdichten so (gering) gewählt sind, dass mit der Biostimulation keine thermischen Wirkungen einhergehen. Um eine derartige Biostimulation geht es bei der vorliegenden Erfindung. In diesem Zusammenhang wird auch der Begriff "Low-Level-Laser" gebraucht.

Es ist bekannt, die genannte Biostimulation zur Bekämpfung der Mucositis einzusetzen. Mucositis, also eine Schleimhautentzündung, kann bei Krebstherapien als Nebenwirkung auftreten, insbesondere bei Einsatz von Cytostatica. Die zur Bekämpfung der Mucositis herkömmlicherweise eingesetzten pharmazeutischen Präparate sind teuer und haben wiederum Nebenwirkungen, sodass in vielen Fällen sich die Biostimulation mit elektromagnetischer Strahlung als vorteilhaft erwiesen hat.

Ganz allgemein betrifft die hier beschriebene Erfindung insbesondere den Einsatz der Biostimulation bei Schleimhautdefekten, insbesondere in der Speiseröhre.

Die DE 10 2006 001 736 A1 beschreibt eine Vorrichtung zum Applizieren von Licht in Körperöffnungen im Sinne der oben genannten "low-level-Therapie". Die Vorrichtung ist distal geschlossen und nicht schlauchförmig. Sie ist auch nicht geeignet zur Einführung in eine Speiseröhre und zur Ernährung des Patienten. Das DE 296 11 520 U1 zeigt eine Vorrichtung, die nicht schlauchförmig ist und auch ungeeignet ist zur Einführung in die Speiseröhre eines Patienten und zu dessen Ernährung.

Der Erfindung liegt die Aufgabe zugrunde, eine medizinische Bestrahlungsvorrichtung der eingangs genannten Art so weiterzubilden, dass sie zur Bekämpfung der Mucositis geeignet ist und gleichzeitig der Ernährung des Patienten dienen kann.

Hierzu stellt die Erfindung eine medizinische Bestrahlungsvorrichtung gemäß dem Anspruch 1 zur Verfügung. Bevorzugte Ausgestaltungen sind in den abhängigen Ansprüchen beschrieben.

Der Begriff "Sonde" beschreibt hier, wie in der Medizintechnik üblich, ein stab-, röhren- oder schlauchförmiges Instrument, das zur Behandlung in zum Beispiel Körperhöhlen eingeführt wird oder auch in die Nähe von zu behandelndem Gewebe gebracht wird. Bei einer stabförmigen Sonde kann der Stab insbesondere elastisch oder plastisch biegsam sein. Als besonders geeignetes Material für die Sonde hat sich erwiesen ein Silikonmaterial oder ein silikonhaltiges Material, welches bezüglich seiner lichtleitenden und lichtdurchlassenden Eigenschaften auf die Bestrahlungsquelle abgestimmt ist. Außer Silikon oder silikonhaltigem Material sind für den Schlauch aber auch PVC und PUR (Polyurethan) geeignet. Allerdings sind Silikonsonden wesentlich weicher und frei von Weichmachern, so dass sie, abhängig vom gewünschten Einsatz, bevorzugt verwendet werden.

Es ist vorgesehen, dass elektromagnetische Strahlung über im Wesentlichen die gesamte Länge der Sonde senkrecht zu dessen Längsachse emittiert wird, wobei die genannte Länge der Sonde, über welche die Strahlung emittiert wird, derjenige Bereich der Sonde ist, der bei bestimmungsgemäßem Gebrauch derselben in Anlage an zu bestrahlendes Gewebe oder in dessen Nähe zu bringen ist. Bereiche der Sonde, die bei bestimmungsgemäßem Gebrauch nicht in die Nähe von zu bestrahlendem Gewebe gebracht werden, brauchen nicht in der beschriebenen Weise Strahlung seitwärts zu emittieren. Dabei ist es auch vorgesehen, eine bereits liegende Sonde (für andere Zwecke) so auszugestalten, dass sie in diesem Zustand (nachträglich) einen Lichtwellenleiter aufnehmen kann, der dann in der vorstehend beschriebenen Weise die Bestrahlung bewirkt.

Insbesondere ist es möglich, bei einer herkömmlichen Sonde (zum Beispiel einer E-Sonde aus Silikon) einen Lichtleiter-Mandrin einzuführen, der entweder über einen

Großteil seiner Längserstreckung oder an seinem distalen Ende Licht emittiert. Dann kann der Lichtleiter in der Sonde hin und her bewegt werden. Hierfür ist es vorteilhaft, die Innenwand der Sonde mit einer die Bewegung des Lichtleiters erleichternden Beschichtung zu versehen.

Es ist vorgesehen, die Sonde sowohl für die genannte Biostimulation durch Strahlung einzusetzen, als auch für die Ernährung eines Patienten bzw. die Zufuhr von Fluid in einen Patienten oder die Abfuhr von Fluid aus einem Patienten.
Die Leistungsdichte bei der hier in Rede stehenden Biostimulation liegt zwischen 1 und 2000 mW/cm².

Eine andere Ausgestaltung der Erfindung sieht vor, dass die Sonde zumindest zwei Lumina (Kanäle) aufweist, wobei in ein Lumen ein Lichtleiter eingeschoben ist. Am proximalen Ende wird in den Lichtleiter elektromagnetische Strahlung eingekoppelt und diese wird über die Längserstreckung des Lichtleiters an gewünschten Stellen sukzessive seitwärts (senkrecht zur Längsachse des Lichtleiters) emittiert und durchdringt die für diese Strahlung transparente Sondenwand, sodass die Sonde insgesamt über zumindest einen Teil, bevorzugt einen Großteil ihrer Längserstreckung seitwärts die für eine Biostimulation geeignete Strahlung abgibt. Die genannte sukzessive Auskopplung von Strahlung seitwärts aus dem Lichtleiter heraus kann zum Beispiel dadurch erfolgen, dass im Lichtleiter eine Vielzahl von Streuzentren angeordnet sind, also Partikel, welche die durch den Lichtleiter laufende elektromagnetische Strahlung seitwärts ablenken und so aus dem Lichtleiter herausführen. Derartige Streuzentren sind als solche in der Lichtleitertechnik bekannt. Es können auch im Lichtleiter eine Vielzahl kleiner Spiegelflächen vorgesehen werden, die die Strahlung, welche von proximal nach distal den Lichtleiter durchläuft, sukzessive seitwärts, also senkrecht zur Lichtleiterachse, umlenken.

Eine andere Ausgestaltung der Erfindung sieht vor, dass die Sonde selbst wie ein Lichtleiter ausgestaltet ist, sodass an ihrem einen Ende (proximal) die für die Biostimulation geeignete elektromagnetische Strahlung eingekoppelt werden kann, welche dann entlang der Längserstreckung der Sonde in dem oben beschriebenen interessierenden Bereich seitwärts ausgekoppelt wird, zum Beispiel über in der Sonde verteilte Licht-Streuzentren.

Eine andere bevorzugte Ausgestaltung der Erfindung sieht vor, dass die Sonde Strahlung mit zumindest zwei unterschiedlichen Wellenlängen emittiert, jeweils bevorzugt monochromatisch zur genannten Biostimulation.

Bevorzugt besteht die Sonde im Wesentlichen aus einem Silikonmaterial oder einem silikonhaltigen Material. Dabei sind Shore-Härten A 50-90, vorzugsweise 60-70 vor gesehen. Im Sondenmaterial wird bevorzugt ein Anteil von BaSO₄ von 10 bis 30% eingearbeitet, vorzugsweise 20%. Im Innenlumen der Sonde wird bevorzugt eine nasschemische Beschichtung vorgesehen.

Die beschriebene Lichtemission (der Begriff "Licht" erfasst hier auch elektromagnetische Strahlung im nicht sichtbaren Bereich) seitwärts über die gesamte Länge der Sonde ist hier bevorzugt eine homogene, also in der Strahlungsdichte gleichmäßige Lichtemission. Das Merkmal "über die gesamte Länge der Sonde" meint Erstreckungslängen, über welche die Strahlung seitwärts aus der Sonde emittiert wird im Zentimeterbereich, länger als 10 cm, je nach medizinischem Einsatz. Die Sonde ist so gestaltet, dass sie bei Einführung in die Speiseröhre über deren gesamte Länge seitwärts, d.h. radial in Bezug auf die Längsachse der Sonde und der Speiseröhre, biostimulierende Strahlung abgibt zur Heilung von insbesondere der Speiseröhrenschleimhaut.
Die vorstehend beschriebenen einzelnen Ausgestaltungen und Merkmale sowie Funktionen der Sonden können sowohl einzeln als auch in beliebiger Kombination der beschriebenen Eigenschaften, Merkmale und Funktionen eingesetzt werden.

Nachfolgend werden Ausführungsbeispiele der Erfindung näher erläutert. Es zeigt:
Fig. 1 schematisch einen Abschnitt eines ersten Ausführungsbeispiels einer Sonde und
Fig. 2 schematisch einen Abschnitt eines zweiten Ausführungsbeispiels einer Sonde.

Die Figuren zeigen jeweils nur einen Abschnitt einer Sonde, soweit es für das Verständnis der Erfindung erforderlich ist.

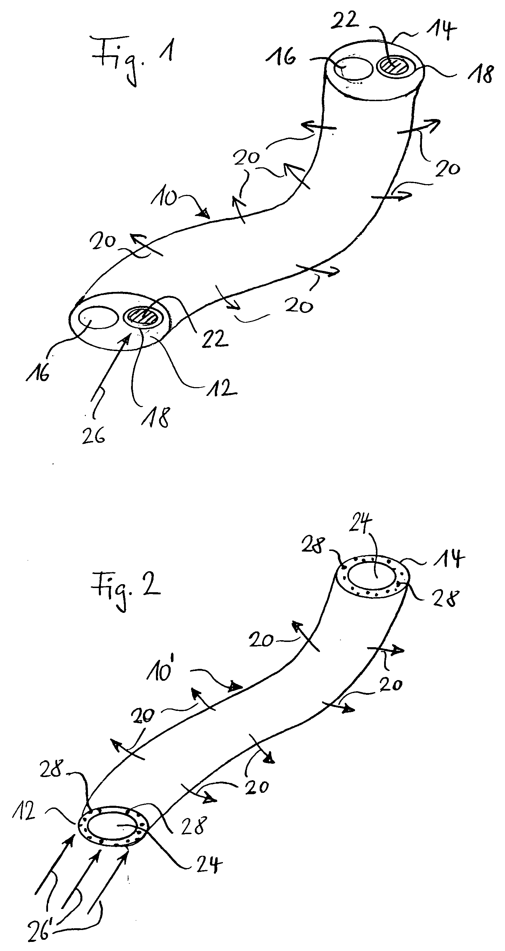

Fig. 1 zeigt als Beispiel eine Sonde 10 für die Ernährung eines Patienten, zum Beispiel eine nasale Sonde oder für die PEG (Percutane Enterale Gastrostomie). Gezeigt ist ein mittlerer Abschnitt der Sonde, es sind also das proximale und das distale Ende der Sonde in der Zeichnung weggelassen. Der gezeigte Abschnitt der Sonde 10 hat ein proximales Ende 12 und ein distales Ende 14. Das distale Ende ist also bei bestimmungsgemäßem Einsatz in der Speiseröhre näher am Magen des Patienten als das andere Ende.

Die Sonde 10 gemäß Fig. 1 hat zwei Lumina 16, 18. In das eine Lumina 18 ist ein Lichtleiter 22 eingeschoben. Am proximalen Ende der Sonde (nicht gezeigt) wird Licht (elektromagnetische Strahlung) in den Lichtleiter 22 eingekoppelt. Das ist in Fig. 1 durch den Pfeil 26 schematisch angedeutet. Der Lichtleiter 22 ist so gestaltet, dass er über seine Längserstreckung sukzessive Licht seitwärts (also quer zu seiner Längsachse) abgibt. Das Material der Sonde 10 ist transparent für diese Strahlung und die somit von der Sonde 10 emittierte Strahlung ist durch die Pfeile 20 in Fig. 1 angedeutet. Zum Beispiel kann der Lichtleiter 22 über seine Längserstreckung verteilt eine Vielzahl von Streuzentren aufweisen, die das Licht seitwärts ablenken. Dabei kann die Dichte der Streuzentren so über die Längserstreckung des Lichtleiters 22 eingestellt werden, dass die seitwärts emittierte Strahlung 20 im Wesentlichen homogen ist, d.h. an jeder Stelle der Längserstreckung der Sonde, über welche die Strahlungsemission erwünscht ist, wird im Wesentlichen pro Flächeneinheit die gleiche Strahlungsmenge abgegeben. Dies kann zum Beispiel dadurch erreicht werden, dass die Dichte der Streuzentren im Lichtleiter über die Längserstreckung des Lichtleiters variiert wird, zum Beispiel derart, dass die Dichte der Streuzentren von proximal nach distal zunimmt.

Das vorstehend beschriebene Ausführungsbeispiel, bei dem der Lichtleiter über seine Längserstreckung sukzessive Licht seitwärts abgibt, kann dahingehend abgewandelt werden, dass der Lichtleiter entweder nur oder auch an seinem distalen Ende elektromagnetische Strahlung insbesondere seitwärts abgibt.

Das nicht von einem Lichtleiter 22 besetzte Lumen 16 der Sonde 10 wird für die Zufuhr oder Abfuhr von Fluid in bzw. aus dem Patienten eingesetzt, für die Ernährung. Es ist auch möglich, das Lumen 16 als Kanal für ein Instrument zu verwenden. Allgemein kann die erfindungsgemäße Sonde auch als Katheter oder als Endoskop gestaltet werden.

Am proximalen Ende (nicht gezeigt) der Sonde 10 gemäß Fig. 1 sind die üblichen Einrichtungen, wie sie aus der Technik der enteralen Ernährung, der Kathetertechnik oder der Endoskopietechnik bekannt sind, angeschlossen, also zum Beispiel Einrichtungen zum Einkoppeln von elektromagnetischer Strahlung in das proximale Ende des Lichtleiters 22 und Einrichtungen für die Zufuhr von Flüssigkeit, im Falle der enteralen Ernährung. Das distale Ende (nicht gezeigt) der in Fig. 1 gezeigten Sonde kann dann ebenfalls in der üblichen Weise, je nach der vorgesehenen Funktion der Sonde, gestaltet sein. Bei dem Einsatz als Ernährungssonde, wäre das distale Ende des Lumens 16 geschlossen und das distale Ende des Lichtleiters 22 könnte entweder freiliegen oder auch verdeckt sein, wenn Strahlung nur seitwärts austreten soll.

Die anhand der Fig. 1 beschriebene Sonde hat insbesondere den Vorteil, dass mit ihr zumindest zwei Funktionen durchgeführt werden können, ohne dass die Sonde neu gelegt werden muss, beim beschriebenen Ausführungsbeispiel ist dies die Ernährung und die Biostimulation.

Analog können mit der anhand der Fig. 1 schematisch beschriebenen Sonde auch andere Funktionen gleichzeitig oder zeitlich versetzt durchgeführt werden, ohne dass die Sonde für die einzelnen Funktionen neu gelegt werden muss, so zum Beispiel kann die Biostimulation kombiniert werden mit der beschriebenen Wund-Drainage. Bei dieser Variante wird proximal am Ende des Lumens 16 eine Saugeinrichtung (Pumpe) angeschlossen, welche am distalen Ende der Sonde anstehende Flüssigkeit und Feuchtigkeit absaugt. Hierzu kann die Sonde auch mit weiteren Öffnungen in der Seitenwand (nicht gezeigt) versehen sein, welche Flüssigkeit in das Lumen 16 aufgrund der Saugwirkung am proximalen Ende eintreten lassen. Hierfür eignet sich besonders eine Zwei-Kanal-Sonde (oder Mehr-Kanal-Sonde).

Bei Kombination der Biostimulation mit einer Evakuierung, zum Beispiel in der oben genannten VAC-Technik, wird ähnlich der vorstehend beschriebenen Drainage, am proximalen Ende der Sonde 10 eine Vakuumpumpe angeschlossen, welche am distalen Ende über das Lumen 16 den Evakuierungseffekt bewirkt.

Die hier anhand der Figuren 1 und 2 sowie der übrigen Beschreibung beschriebenen Sonden sind nicht eingerichtet, um in Gefäßen (Adern) eines Patienten eingesetzt zu werden.

Fig. 2 zeigt eine abgewandelte Ausführungsform einer Sonde. Auch bei dieser Sonde wird die Funktion der Biostimulation mit einer anderen Funktion, die Ernährung eines Patienten, kombiniert. In den Figuren sind einander entsprechende oder funktionsähnliche Komponenten mit gleichen Bezugszeichen versehen, sodass auf eine wiederholte Beschreibung weitgehend verzichtet werden kann.

Die Sonde 10' gemäß Fig. 2 hat ein zentrales Lumen 24. Als Material für die Sonde 10' ist ein lichtleitendes Material gewählt. Ähnlich wie Fig. 1, zeigt Fig. 2 nur einen mittleren Abschnitt der Sonde, wobei das distale Ende und das proximale Ende weggelassen sind. Elektromagnetische Strahlung wird proximal in die Wandung der Sonde 10 eingekoppelt, was in Fig. 2 durch die Pfeile 26' angedeutet ist. Einrichtungen zum Einkoppeln von Licht in einen Lichtleiter sind im Stand der Technik vielfältig bekannt, so dass auf deren Beschreibung hier (wie auch bei Fig. 1) verzichtet werden kann. In der Wand der Sonde 10' sind Streuzentren 28 verteilt, deren Funktion analog der oben anhand der Figur 1 beschriebenen Streuzentren im Lichtleiter 22 ist, d.h. die Streuzentren 28 bewirken, dass die proximal eingekoppelte elektromagnetische Strahlung seitwärts aus der Sonde 10' austritt, was auch hier durch die Pfeile 20 schematisch angedeutet ist. Auch hier sind die Streuzentren in ihrer Dichte so in der Wand der Sonde 10' verteilt, dass die von der Sonde 10' seitwärts abgegebene Strahlung im gewünschten Bereich im Wesentlichen zur Erzielung einer gleichmäßigen Biostimulation homogen austritt.

Für beide Ausführungsbeispiele gemäß den Figuren 1 und 2 sind die Sondenlängen, über welche Strahlung je nach Einsatz austreten kann, oben genannt.

Das Lumen 24 beim Ausführungsbeispiel gemäß Fig. 2 dient für die Ernährung.

Bei beiden Ausführungsbeispielen gemäß den Figuren 1 und 2 können proximal eine Wellenlänge, oder zwei oder mehr unterschiedliche Wellenlängen in die Sonde eingekoppelt werden, wobei aber jede Wellenlänge bevorzugt monochromatisch mit einer Bandbreite von einigen nm eingekoppelt wird. Es kann dann die Biostimulation mit zwei Wellenlängen, jede für sich monochromatisch, durchgeführt werden. Das lässt sich auf mehr als zwei Wellenlängen erweitern. Außer monochromatischem Licht können auch nicht-monochromatische LEDs eingesetzt werden. Auch polarisiertes Licht ist für bestimmte Anwendungen vorteilhaft.

Als Wellenlängen für die Biostimulation kommen Wellenlängen im Bereich von 400 bis 700 nm in Betracht. Die Bandbreite der biostimulierenden Strahlung beträgt bei allen hier beschriebenen Ausführungsbeispielen bevorzugt wenige nm.

Es ist auch möglich, die vorstehend beschriebenen Ausführungsbeispiele von Sonden dahingehend abzuwandeln, dass kein Lumen (Kanal) in der Sonde vorgesehen ist. Zum Beispiel beim Einsatz im Verdauungstrakt (Darm) ist nicht notwendigerweise ein Lumen für einen der oben beschriebenen Zwecke erforderlich. In diesem Falle ist die gesamte Sonde massiv und biegsam zum Beispiel aus lichtleitendem Material mit Streuzentren im oben beschriebenen Sinne versehen. Auch hier gilt für die Strahlungsemission über die Längserstreckung der Sonde das oben gesagte, also insbesondere bezüglich der Strecke der Strahlungsemission, der Homogenität der Strahlung und der Verteilung der Streuzentren zur Erzielung einer solchen Homogenität über die gewünschte Strecke der Sonde.

## Patentansprüche

1. Medizinische Bestrahlungsvorrichtung, adaptiert für eine Biostimulation ohne thermische Wirkung, mit zumindest einer Quelle für elektromagnetische Strahlung im Bereich von 400 bis 700 nm und mit einer Sonde (10), die adaptiert ist, die Strahlung über die gesamte Länge der Sonde (10) zu emittieren, die in Anlage an einen zu bestrahlenden Bereich eines Patienten bringbar ist, **dadurch gekennzeichnet, dass**
die Sonde (10) schlauchförmig und adaptiert ist zur Einführung in die Speiseröhre eines Patienten zu dessen Ernährung, und dass sich die Emission der elektromagnetischen Strahlung seitwärts aus der Sonde über mindestens eine Sondenlänge von 10 cm erstreckt.

2. Medizinische Bestrahlungsvorrichtung nach Anspruch 1, **dadurch gekennzeichnet, dass** die Sonde (10) zumindest zwei Lumina (16, 18) aufweist, wobei in ein Lumen (18) ein Lichtleiter (22) einführbar ist und die Sondenwand für die vom Lichtleiter (22) abgegebene Strahlung (20) durchlässig ist.

3. Vorrichtung nach einem der vorhergehenden Ansprüche, **dadurch gekennzeichnet, dass** die Sonde (10') lichtleitende Eigenschaft hat.

4. Vorrichtung nach einem der vorhergehenden Ansprüche, **dadurch gekennzeichnet, dass** in der Sonde (10') eine Vielzahl von Licht-Streuzentren (28) verteilt sind.

5. Vorrichtung nach einem der vorhergehenden Ansprüche, **dadurch gekennzeichnet, dass** die Sonde Strahlung zumindest zweier Wellenlängen jeweils monochromatisch emittiert.

## Claims

1. A medical radiation device adapted for biostimulation without a thermal effect, having at least one source of electromagnetic radiation in the range of 400 to 700 nm and having a probe (10), which is adapted to emit the radiation over the total length of the probe (10) which can be brought into contact with an area to be irradiated on a patient,
**characterized in that** the probe (10) is in the form of a tube and is adapted for insertion into a patient's esophagus for providing nutrition to said patient, and the emission of the electromagnetic radiation extends laterally out of the probe over a probe length of at least 10 cm.

2. The medical radiation device according to claim 1,
**characterized in that** the probe (10) has at least two lumens (16, 18), wherein a light guide (22) can be inserted into one lumen (18) and the probe wall is permeable for the radiation (20) emitted by the light guide (22).

3. The device according to any one of the preceding claims,
**characterized in that** the probe (10') has light conducting properties.

4. The device according to any one of the preceding claims,
**characterized in that** a plurality of light scattering centers (28) are distributed in the probe (10').

5. The device according to any one of the preceding claims,
**characterized in that** the probe emits monochromatic radiation of at least two wavelengths in each case.

## Revendications

1. Dispositif pour rayonnement médical, conçu pour une bio-stimulation sans effet thermique et comprenant au moins une source de rayonnement électromagnétique d'une longueur d'onde comprise entre 400 à 700 nm et une sonde (10) qui est conçue pour émettre le rayonnement sur toute la longueur de la sonde (10) et peut être mise en application avec une région du patient à irradier, **caractérisé en ce que**
la sonde (10) se présente sous la forme d'un tube souple et qu'elle est conçue pour être introduite dans l'oesophage d'un patient à alimenter par sonde, et **en ce que** l'émission du rayonnement électromagnétique s'opère latéralement sur au moins une longueur de sonde de 10 cm.

2. Dispositif pour rayonnement médical selon la revendication 1, **caractérisé en ce que** la sonde (10) présente au moins deux lumières (16, 18), un guide de lumière (22) pouvant être introduit dans une lumière (18) et la paroi de la sonde étant perméable au rayonnement (20) émis par le guide de lumière (22).

3. Dispositif pour rayonnement médical selon l'une des revendications précédentes, **caractérisé en ce que** la sonde (10') a la propriété de conduire la lumière.

4. Dispositif pour rayonnement médical selon l'une des revendications précédentes, **caractérisé en ce qu'**une pluralité de centres de diffusion de lumière (28) est répartie à l'intérieur de la sonde (10').

5. Dispositif pour rayonnement médical selon l'une des revendications précédentes, **caractérisé en ce que** la sonde émet un rayonnement selon au moins deux longueurs d'ondes monochromatiques.
